# EUROPEAN PATENT APPLICATION

(11) **EP 2 620 154 A1**
(43) Date of publication of application: **31.07.2013**
(21) Application number: 11826917.4
(22) Date of filing: 22.09.2011
(51) Int. Cl.: A61K 31/661, A61K 31/685, A61P 21/00, A61P 25/00, A61P 25/16, A61P 25/18, A61P 25/24, A61P 25/28

(54) **DRUG AGAINST CENTRAL NERVOUS SYSTEM INFLAMMATION**

(30) Priority: 24.09.2010 JP 2010214358
(71) Applicant: Fujino Brain Research Co., Ltd., Tokyo 104-0061 (JP); Marudai Food Co., Ltd., Osaka 569-8577 (JP); Umeda Jimusho Ltd., Tokyo 151-0053 (JP)
(72) Inventor: MAWATARI, Shirou, Kasuya-gun Fukuoka 811-2501 (JP); FUJINO, Takehiko, Fukuoka-shi Fukuoka 812-0025 (JP); IFUKU, Masataka, Fukuoka-shi Fukuoka 812-8582 (JP); SUGIYAMA, Masaaki, Takatsuki-shi Osaka 569-8577 (JP); FUCHU, Hidetaka, Takatsuki-shi Osaka 569-8577 (JP)
(74) Representative: Müller-Boré & Partner Patentanwälte
(86) International application number: PCT/JP2011/071681
(87) International publication number: WO 2012/039472

(57) **Abstract**

An objective of the present invention is to provide a novel method with an effect of alleviating central nervous system inflammation. The present invention provides a drug against central nervous system inflammation containing a plasmalogen. More preferably, the present invention provides a drug against central nervous system inflammation containing a plasmalogen extracted from a biological tissue (preferably an avian tissue) that mainly contains an ethanolamine plasmalogen and a choline plasmalogen.

## Description

### Technical Field

The present invention relates to a drug against central nervous system inflammation containing a plasmalogen. More specifically, the present invention relates to a drug against central nervous system inflammation containing, as an active ingredient, a plasmalogen extracted from a biological tissue. The drug against central nervous system inflammation may also be referred to as a drug for preventing and/or treating central nervous system inflammation.

### Background Art

The central nervous system is an assembly of many nerve cells, such as brain, spinal cord, etc., in vertebrates. These nerve cells are covered and protected by encephalon or meninges. There are many causes of central nerve inflammation (central nervous system inflammation: encephalitis, myelitis, meningitis, etc.). For example, acute or chronic encephalitis, myelitis, meningitis, etc., may be caused by fungus, bacteria, or virus infection. Further, leukemia or malignant brain tumor may also induce inflammation.

The central nervous system inflammation is known to damage nerve cells, and thereby causes various diseases and disorders. For example, when inflammation due to bacterial or viral encephalitis significantly damages the brain, it may result in frequent occurrence of confusion, spasm seizure, and coma depending on the type of bacteria or virus; in the worse-case scenario, it may even result in death. Further, even if the patient recovers, the patient is likely to have an eternal nerve disorder due to nerve cell damage.

Recent studies revealed that most chronic neurodegenerative diseases, such as Alzheimer's disease (AD), Parkinson's disease, amyotrophic lateral sclerosis (ALS), or multiple sclerosis, or acute brain damages such as cerebral apoplexy or head injury often induce chronic central nerve (brain, spinal cord, etc.) inflammation. Further, there is also an assumption that these diseases are actually caused and advanced by central inflammation. For example, some reports suggest that central nervous system inflammation causes or advances neurodegenerative diseases such as Alzheimer's disease (e.g. Non-Patent Document 1). Another document reports an analysis of a rat with memory loss intraperitoneally administered with LPS (lipopolysaccharide), which is an inflammation-inducing substance. The report revealed that there was accumulation of Aβ (amyloid beta) peptide in the rat brain, and that the symptom was relieved by sulindac sulfide, serving as an anti-inflammatory agent (Non-Patent Document 2).

It has also been reported that mental or developmental disorder such as depression or autism, or even normal aging result in a higher rate of central nervous system inflammation.

Accordingly, there is an increasing expectation that preventing or treating central nervous system inflammation can prevent neurological disorder due to nervous cell damage caused by infectious inflammation; or can treat neurodegenerative diseases such as Alzheimer's disease or Parkinson's disease, or mental or developmental disorders such as schizophrenia, depression, or autism.

Under such present circumstances, demand for a method of effectively treating central nervous system inflammation without causing side effects is further increasing.

### Citation List

### Non-Patent Documents

NPD 1: Yan Q et al., J Neurosci. 2003 Aug 20; 23(20):7504-9
NPD 2: Lee JW et al., Neuroinflammation. 2008 Aug 29; 5:37.

### Summary of Invention

### Technical Problem

An objective of the present invention is to provide a novel drug having an effect of treating and/or preventing central nervous system inflammation.

### Solution to Problem

Surprisingly, the present inventors have discovered that a plasmalogen suppresses proliferation of glial cells, which is considered one of the causes of central nervous system inflammation, and that a plasmalogen thereby treats central nervous system inflammation. Based on this discovery, the present inventors conducted further studies, and accomplished the present invention.

Specifically, the present invention encompasses, for example, the subjects described in the following items.
Item 1.
   A drug against central nervous system inflammation containing a plasmalogen.
Item 2.
   The drug against central nervous system inflammation according to Item 1, containing a plasmalogen extracted from a biological tissue.
Item 3.
   The drug against central nervous system inflammation according to Item 2, containing a plasmalogen extracted from an avian tissue.
Item 4.
   The drug against central nervous system inflammation according to any one of Items 1 to 3, wherein the plasmalogen includes an ethanolamine plasmalogen and a choline plasmalogen.
Item 5.
   The drug against central nervous system inflammation according to Item 4, wherein 90 mass% or more of the plasmalogen is an ethanolamine plasmalogen and a choline plasmalogen.
Item 6.
   The drug against central nervous system inflammation according to Item 4 or 5, wherein a mass ratio of ethanolamine plasmalogen:choline plasmalogen in the plasmalogen is 1:5 to 5:1.
Item 7.
   The drug against central nervous system inflammation according to any one of Items 1 to 6, utilized for preventing or treating at least one type of disease selected from the group consisting of dementia (in particular, Alzheimer's disease), Parkinson's disease, depression, and schizophrenia.
Item 8.
   The drug against central nervous system inflammation according to any one of Items 3 to 7, wherein the plasmalogen is produced through a method comprising the steps of:
   (1) subjecting bird skin to extraction using ethanol or hydrous ethanol;
   (2) recovering a precipitate after centrifugation from the extract obtained in Step (1) using acetone, and recovering a liquid layer after centrifugation using a mixed solvent of hexane and acetone; and
   (3) treating the liquid obtained in Step (2) using phospholipase A1(PLA1).
Item 9.
   A method for treating central nervous system inflammation, comprising oral administration or intravascular administration of a plasmalogen.
Item 10.
   The method for treating central nervous system inflammation according to Item 9, comprising the oral administration or intravascular administration of a plasmalogen extracted from a biological tissue (preferably an avian tissue).
Item 11.
   The method for treating central nervous system inflammation according to Item 9 or 10, wherein the plasmalogen includes an ethanolamine plasmalogen and a choline plasmalogen (preferably 90 mass% or more of the plasmalogen is an ethanolamine plasmalogen and a choline plasmalogen).
Item 12.
   The method for treating central nervous system inflammation according to Item 11, wherein a mass ratio of ethanolamine plasmalogen:choline plasmalogen in the plasmalogen is 1:5 to 5:1.
Item 13.
   The method for treating central nervous system inflammation according to any one of Items 9 to 12, wherein the method is to treat a disease selected from the group consisting of dementia (in particular, Alzheimer's disease), Parkinson's disease, depression, and schizophrenia.
Item 14.
   The method for treating central nervous system inflammation according to any one of Items 10 to 13, wherein the plasmalogen is produced through a method comprising the steps of:
   (1) subjecting bird skin to extraction using ethanol or hydrous ethanol;
   (2) recovering a precipitate after centrifugation from the extract obtained in Step (1) using acetone, and recovering a liquid layer after centrifugation using a mixed solvent of hexane and acetone; and
   (3) treating the liquid obtained in Step (2) using phospholipase A1(PLA1).
Item 15.
   A plasmalogen used for the treatment of central nervous system inflammation.
Item 16.
   The plasmalogen according to Item 15, wherein the plasmalogen is extracted from a biological tissue (preferably an avian tissue).
Item 17.
   The plasmalogen according to Item 15 or 16, wherein the plasmalogen includes an ethanolamine plasmalogen and a choline plasmalogen (preferably 90 mass% or more of the plasmalogen is an ethanolamine plasmalogen and a choline plasmalogen).
Item 18.
   The plasmalogen according to Item 16 or 17, wherein the plasmalogen is produced through a method comprising the steps of:
   (1) subjecting bird skin to extraction using ethanol or hydrous ethanol;
   (2) recovering a precipitate after centrifugation from the extract obtained in Step (1) using acetone, and recovering a liquid layer after centrifugation using a mixed solvent of hexane and acetone; and
   (3) treating the liquid obtained in Step (2) using phospholipase A1(PLA1).
Item 19.
   Use of plasmalogen for the production of a medication for central nervous system inflammation.
Item 20.
   The use according to Item 19, wherein the plasmalogen is extracted from a biological tissue (preferably an avian tissue).
Item 21.
   The use according to Item 19 or 20, wherein the plasmalogen includes an ethanolamine plasmalogen and a choline plasmalogen (preferably 90 mass% or more of the plasmalogen is an ethanolamine plasmalogen and a choline plasmalogen).
Item 22.
   The use according to any one of Items 19 to 21, wherein the plasmalogen is produced through a method comprising the steps of:
   (1) subjecting bird skin to extraction using ethanol or hydrous ethanol;
   (2) recovering a precipitate after centrifugation from the extract obtained in Step (1) using acetone, and recovering a liquid layer after centrifugation using a mixed solvent of hexane and acetone; and
   (3) treating the liquid obtained in Step (2) using phospholipase A1(PLA1).

Furthermore, the present invention encompasses, for example, a drug or a method for inhibiting glial cell proliferation defined in the following Items A-1 to M.
Item A-1.
   An inhibitor of glial cell proliferation containing a plasmalogen.
Item A-2.
   An inhibitor of glial cell proliferation associated with central nervous system inflammation, the inhibitor comprising a plasmalogen.
Item B.
   The inhibitor of glial cell proliferation according to Item A-1 or A-2, the inhibitor comprising a plasmalogen extracted from a biological tissue.
Item C.
   The inhibitor of glial cell proliferation according to Item B, the inhibitor comprising a plasmalogen extracted from an avian tissue.
Item D.
   The inhibitor of glial cell proliferation according to any one of Items A-1 to C, wherein the plasmalogen includes an ethanolamine plasmalogen and a choline plasmalogen.
Item E.
   The inhibitor of glial cell proliferation according to Item D, wherein 90 mass% or more of the plasmalogen is an ethanolamine plasmalogen and a choline plasmalogen.
Item F.
   The inhibitor of glial cell proliferation according to Item D or E, wherein a mass ratio of ethanolamine plasmalogen:choline plasmalogen in the plasmalogen is 1:5 to 5:1.
Item G.
   The inhibitor of glial cell proliferation according to Items A-1 to F, wherein the method is to treat a disease selected from the group consisting of dementia (in particular, Alzheimer's disease), Parkinson's disease, depression, and schizophrenia.
Item H.
   The inhibitor of glial cell proliferation according to any one of Items C to G, wherein the plasmalogen is produced through a method comprising the steps of:
   (1) subjecting bird skin to extraction using ethanol or hydrous ethanol;
   (2) recovering a precipitate after centrifugation from the extract obtained in Step (1) using acetone, and recovering a liquid layer after centrifugation using a mixed solvent of hexane and acetone; and
   (3) treating the liquid obtained in Step (2) using phospholipase A1(PLA1).
Item I.
   A glial cell proliferation inhibition method, comprising oral administration or intravascular administration of an effective dose of a plasmalogen.
Item J.
   The glial cell proliferation inhibition method according to Item I, comprising oral administration or intravascular administration of a plasmalogen extracted from a biological tissue (preferably an avian tissue).
Item K.
   The glial cell proliferation inhibition method according to Item I or J, wherein the plasmalogen includes an ethanolamine plasmalogen and a choline plasmalogen (preferably 90 mass% or more of the plasmalogen is an ethanolamine plasmalogen and a choline plasmalogen).
Item L.
   The glial cell proliferation inhibition method according to Item K, wherein a mass ratio of ethanolamine plasmalogen:choline plasmalogen in the plasmalogen is 1:5 to 5:1.
Item M.
   The glial cell proliferation inhibition method according to any one of Items I to K, wherein the plasmalogen is produced through a method comprising the steps of:
   (1) subjecting bird skin to extraction using ethanol or hydrous ethanol;
   (2) recovering a precipitate after centrifugation from the extract obtained in Step (1) using acetone, and recovering a liquid layer after centrifugation using a mixed solvent of hexane and acetone; and
   (3) treating the liquid obtained in Step (2) using phospholipase A1(PLA1).

### Advantageous Effects of Invention

The drug against central nervous system inflammation according to the present invention containing a plasmalogen is capable of alleviating and treating central nerve inflammation. It is assumed that release of inflammatory cytokine from activated glial cells in the central nerve system (brain, spinal cord, etc.) is one of the causes of central nervous system inflammation (e.g., encephalitis, meningitis). The drug against central nervous system inflammation of the present invention has an effect of inhibiting proliferation of glial cells that are proliferated and activated by central nerve inflammation. With this effect, the drug of the present invention alleviates and treats central nervous system inflammation. More specifically, administration of the drug against central nervous system inflammation of the present invention suppresses proliferation of glial cells in the inflammatory site, thereby alleviating and treating inflammation. Accordingly, the drug against central nervous system inflammation of the present invention may also be considered to be a glial cell proliferation inhibitor.

Moreover, as mentioned above, the drug against central nervous system inflammation of the present invention may also be preferably used for treatment of diseases that are considered to partially derive from central nerve inflammation, such as chronic neurodegenerative diseases including dementia (in particular, Alzheimer's disease (AD)), Parkinson's disease, amyotrophic lateral sclerosis (ALS), multiple sclerosis, and mental disorders including schizophrenia, depression, and autism.

Biological tissues generally contain a large amount of plasmalogen. Biological tissues containing plasmalogen have hitherto been used as food. Therefore, the drug against central nervous system inflammation of the present invention containing a plasmalogen (in particular, a plasmalogen extracted from a biological tissue) is considered to have few side effects, and thereby ensures high safety.

### Brief Description of Drawings

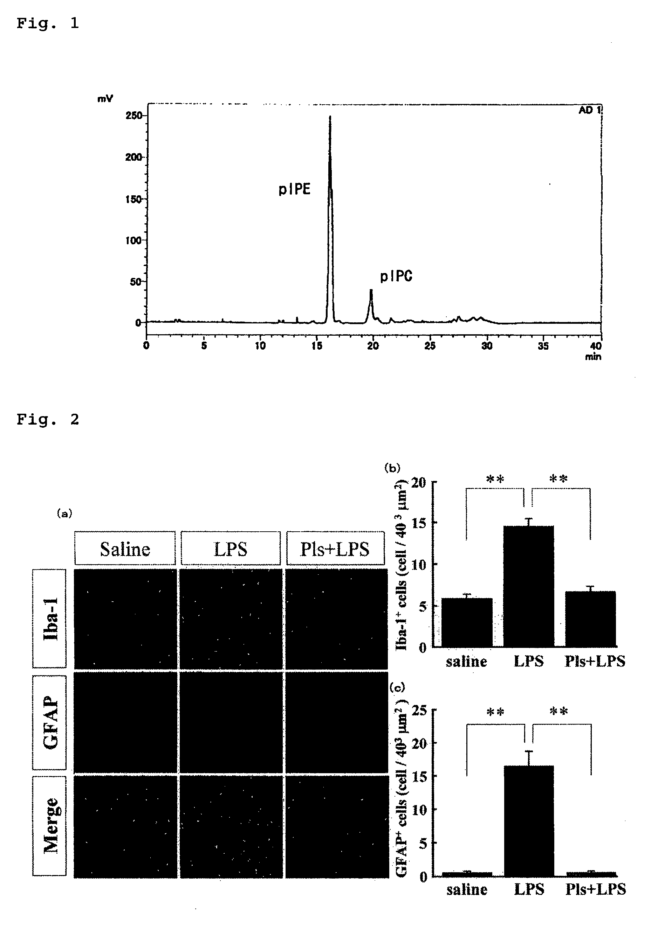

Fig. 1 shows a chromatogram obtained through analysis of a highly pure plasmalogen-containing substance extracted from chicken skin, using HPLC-ELSD. "plPE" represents ethanolamine plasmalogens, and "plPC" represents choline plasmalogens.
Fig. 2 shows the results of immunostaining of cerebral (cerebral cortex) segments of mice intraperitoneally administered with either a physiological saline solution, LPS, or a plasmalogen and LPS (LPS+Pls), using an anti-Iba-1 antibody or an anti-GFAP antibody. Fig. 2(a) shows images obtained by a confocal microscope, as well as a merged image, and Fig. 2(b) and Fig. 2(c) show results of analysis of glial cell count of the images. Fig. 2(b) shows an analysis of microglial cell count, and Fig. 2(c) shows an analysis of astrocyte cell count.

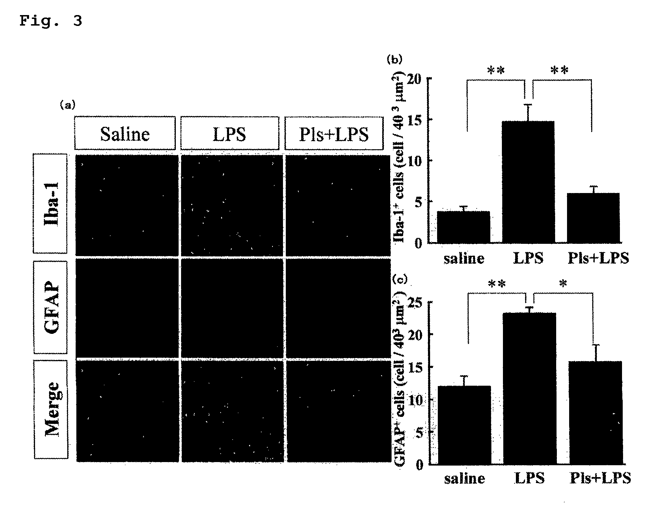

Fig. 3 shows the results of immunostaining of cerebral (dentate gyrus) segments of mice intraperitoneally administered with either a physiological saline solution, LPS, or a plasmalogen and LPS (LPS+Pls), using an anti-Iba-1 antibody or an anti-GFAP antibody. Fig. 3(a) shows images obtained by a confocal microscope, as well as a merged image, and Fig. 3(b) and Fig. 3(c) show results of analysis of glial cell count of the images. Fig. 3(b) shows an analysis of microglial cell count, and Fig. 3(c) shows an analysis of astrocyte cell count.

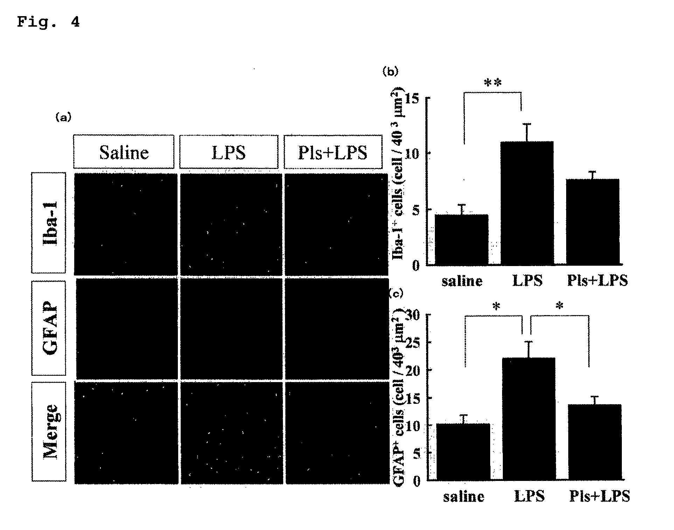

Fig. 4 shows the results of immunostaining of cerebral (hippocampus CA1) segments of mice intraperitoneally administered with either a physiological saline solution, LPS, or a plasmalogen and LPS (LPS+Pls), using an anti-Iba-1 antibody or an anti-GFAP antibody. Fig. 4(a) shows images obtained by a confocal microscope, as well as a merged image, and Fig. 4(b) and Fig. 4(c) show results of analysis of glial cell count of the images. Fig. 4(b) shows an analysis of microglial cell count, and Fig. 4(c) shows an analysis of astrocyte cell count.

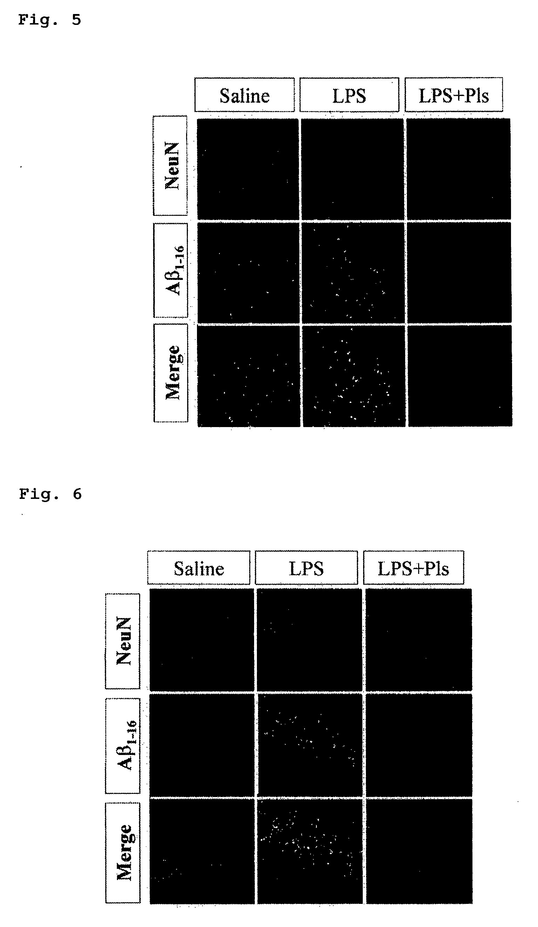

Fig. 5 shows the results of immunostaining of cerebral (cerebral cortex) segments of mice intraperitoneally administered with either a physiological saline solution, LPS, or a plasmalogen and LPS (LPS+Pls), using an anti-NeuN antibody or an anti-Aβ (amyloid beta) antibody. Fig. 5 shows images obtained by a confocal microscope, as well as a merged image.
Fig. 6 shows the results of immunostaining of cerebral (hippocampus CA1) segments of mice intraperitoneally administered with either a physiological saline solution, LPS, or a plasmalogen and LPS (LPS+Pls), using an anti-NeuN antibody or an anti-Aβ (amyloid beta) antibody. Fig. 6 shows images obtained by a confocal microscope, as well as a merged image.

### Description of Embodiments

The present invention is described below in more detail.

The present invention relates to a drug against central nervous system inflammation containing a plasmalogen.

A plasmalogen normally refers to a glycerophospholipid having a long-chain alkenyl group at position 1 of the glycerol skeleton via a vinyl ether linkage. A general formula of a plasmalogen is shown below.

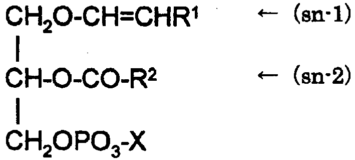

In the formula, R¹ and R² each represent an aliphatic hydrocarbon group. R¹ is generally an aliphatic hydrocarbon group having a carbon number of 1 to 20. Examples thereof include dodecyl group, tetradecyl group, hexadecyl group, octadecyl group, icosanyl group, and the like. R² is generally an aliphatic hydrocarbon group derived from a fatty acid residue. Examples thereof include octadecadienoyl group, octadecatrienoyl group, icosatetraenoyl group, icosapentaenoyl group, docosatetraenoyl group, docosapentaenoyl group, docosahexaenoyl group, and the like. In the formula, X represents a polar group. X is preferably ethanolamine, choline, serine, inositol, or glycerol. Plasmalogens widely present in nature are, in particular, an ethanolamine plasmalogen of the above formula wherein X is ethanolamine, and a choline plasmalogen of the above formula wherein X is choline.

Plasmalogens used in the present invention are preferably extracted from biological tissues. Here, a biological tissue is a tissue containing a plasmalogen in an organism. Examples of organisms used for extracting plasmalogens include animals and microorganisms. As microorganisms, anaerobic bacteria are preferable, and, for example, bacteria in the family Acidaminococcaceae, which are intestinal bacteria, are particularly preferable. In terms of bacteria, "biological tissue" refers to bacteria themselves. Suitable animals include birds, mammals, fishes, shellfishes, and the like. As mammals, livestock are preferable, considering both aspects of stable supply and safety. Examples thereof include cow, pig, horse, sheep, goat, and the like. In terms of mammals, examples of tissues containing a plasmalogen include skin, brain, intestines, heart, genitals, and the like. Plasmalogens can be extracted from these tissues. Examples of birds include chicken, domestic duck, quail, duck, pheasant, ostrich, turkey, and the like. Considering the availability, cost, reluctance to eating, etc., chicken is particularly preferable. There is no particular limitation to an avian tissue to be used. For example, bird meat (in particular, bird's breast meat), bird skin, internal organs of birds, etc., are preferably used. Two or more types of different tissues from one or more species of organisms may be used in combination.

In the present invention, a plasmalogen extracted from an avian tissue is particularly preferably used as the plasmalogen extracted from a biological tissue. Of these, birds (poultry), which have been conventionally used as food, are preferable, as they are proven to be safe and are easily supplied in a stable manner. Chicken is the most preferable.

The method for extracting a plasmalogen from a biological tissue is not limited, as long as a plasmalogen can be extracted (and purified, if necessary). From the viewpoints of convenience, cost, and the like, it is preferable that extraction and purification be performed in the following manner. This method for extraction and purification is preferable, because diacyl glycerophospholipids can be degraded/removed, and the purity of plasmalogen can thereby be further increased.

Specifically, extraction and purification of a plasmalogen can be performed by a method comprising the steps of:
(1) extracting a plasmalogen from a biological tissue;
(2) purifying the plasmalogen in the extract (specifically, removing neutral lipids and/or sphingolipids); and
(3) purifying the extract after hydrolysis treatment (specifically, hydrolyzing diacyl glycerophospholipids, and subsequently removing free fatty acids and lysophospholipids). Here, Step (1) above is a step of plasmalogen extraction, and Steps (2) and (3) are steps of purification. Therefore, Steps (2) and (3) are arbitrary steps, and may not be included in this method; however, it is preferable to use a plasmalogen that is concentrated by purification. In particular, a drug against central nervous system inflammation containing a plasmalogen that is extracted and purified by a method comprising all of Steps (1) to (3) is preferable as it exerts more excellent effects.

Hereinafter, a "plasmalogen extracted from a biological tissue" may be described as a "biological-tissue extracted plasmalogen." Further, for example, a "plasmalogen extracted from an avian tissue" may be described as an "avian-tissue extracted plasmalogen."

Extraction is preferably conducted through an extraction using water or an organic solvent (e.g., methanol, ethanol, propanol, butanol, isopropanol, acetone, or hexane; or a solvent mixture comprising at least two organic solvents selected from the group consisting of these organic solvents), or a hydrous organic solvent. The water content in the hydrous organic solvent is not particularly limited, and is, for example, 10 to 90%. In particular, extraction using ethanol or hydrous ethanol is preferable. Furthermore, an avian tissue on which the extraction is to be conducted may be raw or pre-processed in some manner. For example, the avian tissue may be dried and/or deoiled in advance.

There is no particular limitation to extraction conditions, and an immersion method, such as cold extraction or warm extraction, a percolation method, or the like may be used. One suitable example is a method comprising adding ethanol to chicken skin, and keeping the mixture still or stirring the mixture at 30°C or higher for 60 minutes or more, preferably at 40°C or higher for 180 minutes or more. This method can be performed by using, for example, 1 to 10 L, preferably 1 to 6 L, and more preferably 2 to 4 L, of ethanol with respect to, for example, 1 kg of dried, deoiled chicken skin.

The obtained organic solvent extraction solution is preferably condensed, and is more preferably condensed and dried into a solid. Condensation (or condensation to dryness) can be conducted in accordance with a method known in the art, and can be conducted, for example, using an evaporator. The organic solvent extraction solution is condensed and dried into a solid to obtain a dry solid organic solvent extraction. Lipids such as plasmalogens are contained in a condensed manner in the dry solid organic solvent extraction.

Further, the dry solid organic solvent extraction is preferably, for example, centrifuged with acetone to recover a precipitate, and more preferably is further centrifuged with a mixed solvent of hexane and acetone to recover a liquid layer. Although a restrictive interpretation is not desired, neutral lipids can be removed by recovering a precipitate after centrifugation using acetone, and sphingolipids can be removed by recovering a liquid layer after centrifugation using a mixed solvent of hexane and acetone.

The thus-obtained liquid layer is condensed and dried into a solid to obtain a condensed dry solid phospholipid. The condensed dry solid phospholipid is subjected to a hydrolysis treatment step to hydrolyze diacyl phospholipids to thereby desirably condense the plasmalogen.

Examples of such hydrolysis treatment include treatment using phospholipase A1 (PLA1). PLA1 specifically hydrolyzes the linkage in a diacyl phospholipid between the fatty acid at the sn-1 position and the glycerin skeleton. In contrast, a plasmalogen contains an ether linkage at the sn-1 position, and is therefore not subjected to the action of PLA1. Therefore, treatment using PLA1 degrades diacyl glycerophospholipids, but not plasmalogens. Treatment using PLA1 degrades diacyl glycerophospholipids into free fatty acids and lysophospholipids. By converting diacyl glycerophospholipids that coexist with plasmalogens into the lyso form using PLA1, and removing free fatty acids and lysophospholipids, plasmalogens can be purified. The free fatty acids and lysophospholipids can be removed, for example, by partitioning with acetone and hexane.

With regard to PLA1, there is no particular limitation to its origin, etc., as long as the above-described advantageous effect can be obtained. Examples thereof include PLA1 derived from *Aspergillus oryzae.* Such PLA1 can be purchased from, for example, Mitsubishi-Kagaku Foods Corporation, or the like. The usage amount thereof can be set as appropriate in accordance with the amount of the dry solid organic solvent extraction to be used. It is preferable that PLA1 be used in an amount of 0.2 to 200 units/(1 mg of the dry solid organic solvent extraction), and more preferably 2 to 200 unit/(1 mg of the dry solid organic solvent extraction). 1 unit refers to an amount (1 µmol/min) that transforms 1 µmol of the substrate (diacyl glycerophospholipid) in 1 minute.

A buffer to be used can also be selected as appropriate in accordance with the PLA1 to be used. For example, a 0.1 M citric acid-HCl buffer (pH: 4.5) can be used. In such a case, the buffer is added to dissolve the dry solid organic solvent extraction, after which PLA1 may be added thereto. The amount of the buffer that is to be used is not particularly limited as long as the enzyme reaction can proceed, and is preferably 1 to 30 mL, and more preferably about 5 to 15 mL, per 1 g of the dry solid organic solvent extraction.

The reaction conditions can also be set as appropriate; the reaction is preferably conducted at 50°C for 1 to 2 hours while stirring.

An inactivation treatment may be conducted on the enzyme. This treatment is preferably conducted after the hydrolysis reaction by increasing the temperature to about 70°C.

In the manner described above, a treatment liquid (hydrolysis treatment liquid) in which diacyl glycerophospholipids are degraded can be obtained. By adding, to the hydrolysis treatment liquid, hexane at a volume of, for example, 2 to 3 times that of the hydrolysis treatment liquid, centrifuging the resulting mixture, and recovering a liquid layer, the enzyme buffer and enzyme protein can be removed.

Additionally, plasmalogens are soluble to hexane, but poorly soluble to acetone. Therefore, by performing partitioning with an arbitrary combination of these solvents and water, and by further performing partitioning with water or an aqueous solution, lysophospholipids can be removed and plasmalogens can be purified. Specifically, the use of acetone enables removal of neutral lipids other than phospholipids, and partitioning with a water-based solution can separate plasmalogens from lysophospholipids.

As is clear from the above, for example, Steps (1) to (3) can be described in further detail as follows:
(1) subjecting bird skin to extraction using ethanol or hydrous ethanol;
(2) recovering a precipitate after centrifugation from the extract obtained in Step (1) using acetone, and recovering a liquid layer after centrifugation using a mixed solvent of hexane and acetone; and
(3) treating the liquid obtained in Step (2) using phospholipase A1(PLA1) (and, if necessary, a step of removing free fatty acids and lysophospholipids by partitioning with acetone and hexane). The biological tissue-extracted plasmalogen that is extracted and purified, for example, in this manner can be preferably used as an active ingredient of the drug against central nervous system inflammation of the present invention.

The biological tissue-extracted plasmalogen mainly includes an ethanolamine plasmalogen and/or a choline plasmalogen (i.e., includes at least one member selected from the group consisting of an ethanolamine plasmalogen and a choline plasmalogen). The mass ratio of ethanolamine plasmalogen to choline plasmalogen in the biological tissue-extracted plasmalogen (ethanolamine plasmalogen:choline plasmalogen) is preferably about 1:5 to 1:0, more preferably about 1:5 to 5:1, still more preferably about 1:3 to 3:1, even more preferably about 1:1 to 3:1, and most preferably about 1:1 to 2:1.

The biological tissue-extracted plasmalogen used in the present invention includes an ethanolamine plasmalogen and a choline plasmalogen at a concentration of, on a dry mass basis, preferably 50 mass% or more, more preferably 60 mass% or more, still more preferably 70 mass% or more, even more preferably 80 mass% or more, yet even more preferably 90 mass% or more, and particularly preferably 92 mass% or more.

The mass ratio of ethanolamine plasmalogen to choline plasmalogen and the amounts thereof can be obtained, for example, by analyzing the biological tissue-extracted plasmalogen using high-performance liquid chromatography (HPLC). Specifically, the mass ratio can be obtained with HPLC by obtaining a chromatogram using an evaporative light scattering detector (ELSD) (i.e., using HPLC-ELSD), and obtaining an area ratio of respective peaks representing the ethanolamine plasmalogen and the choline plasmalogen in the chromatogram. The amount can be obtained by calculating what % of the peak area of the entire chromatogram is the peak area representing the ethanolamine plasmalogen or the choline plasmalogen. This is because, with ELSD, substances having similar structures show a similar area response. Since a choline type is electrically neutral, whereas ethanolamine is weakly acidic due to the negative charge of phosphate, the analysis is conducted after, for example, charging acidic lipids using acetic acid and triethylamine as solvents. This is because a more similar area response can be obtained with the charging.

The drug against central nervous system inflammation of the present invention is preferably used in the pharmaceutical field and food field. The agent includes a plasmalogen (preferably, the biological tissue-extracted plasmalogen).

The drug against central nervous system inflammation of the present invention can be preferably used for preventing, treating, or alleviating central nervous system inflammation. Examples of central nervous system inflammation include encephalitis and meningitis. The drug against central nervous system inflammation of the present invention is effective, in particular, for central nervous system inflammation accompanied with an increase in glial cells.

When the drug against central nervous system inflammation of the present invention is used in the pharmaceutical field, the drug (hereinafter, sometimes referred to as "pharmaceutical agent of the present invention") may consist only of plasmalogen, or may contain other components (i.e., may be a pharmaceutical composition containing a plasmalogen). For example, in the pharmaceutical agent of the present invention, it is possible to incorporate, into a plasmalogen, which is an active ingredient, pharmaceutically acceptable bases, carriers, additives (e.g., excipients, binders, disintegrants, lubricants, solvents, sweetening agents, coloring agents, corrigents, odor-masking agents, surfactants, moisturizers, preservatives, pH adjusters, and thickening agents), and the like, if necessary. Such bases, carriers, additives, etc., are specifically described, for example, in Japanese Pharmaceutical Excipients Directory 2000 (Yakuji Nippo Limited), and, for example, those mentioned therein may be used. The drug of the present invention may be formed into a preparation form, such as tablets, coated tablets, powders, granules, subtle granules, capsules, pills, liquid agents, suspensions, emulsions, jellies, chewable agents, soft tablets, etc., by using a hitherto known method. In particular, the drug of the present invention is preferably formed into a liquid agent, a suspension, an emulsion, or the like, and used as an injection or drops. The use thereof as an oral preparation is also possible.

The amount of plasmalogen contained in the pharmaceutical agent of the present invention is not particularly limited as long as the central nervous system inflammation prevention effect is exerted, and can be determined as appropriate according to a preferable plasmalogen intake amount per day. The amount is preferably 0.0005 to 100 mass%, more preferably 0.005 to 90 mass%, and still more preferably 0.05 to 80 mass%.

A subject to which the pharmaceutical agent of the present invention is administered is preferably the one who suffers from central nervous system inflammation. Examples thereof include subjects developing central nervous system inflammation due to infection with fungus, bacteria, or virus.

It is preferable that the subject suffer from a disease in which one of the causes is assumed to be central nervous system inflammation. Examples of such diseases include neurodegenerative diseases, mental disorders, and the like. Specific examples of neurodegenerative diseases include Alzheimer's disease (AD), Parkinson's disease, amyotrophic lateral sclerosis (ALS), multiple sclerosis, and the like. Specific examples of mental disorders include depressive psychosis, manic psychosis, manic-depressive psychosis, schizophrenia, autism, eating disorder, and the like. Further, the pharmaceutical agent of the present invention may also be prophylactically administered to subjects that have a high future possibility of developing these diseases. For example, the pharmaceutical agent of the present invention may be prophylactically administered to subjects that are shown genetically to have a high possibility of developing the diseases exemplified above, or to elderly people (in particular, at the age of 60 or over).

The subject to which the pharmaceutical agent of the present invention is administered is not necessarily a human, and may be a mammal other than a human. Such mammals may be, for example, those that are reared as pets or livestock. Examples thereof include dogs, cats, cows, horses, pigs, sheep, goats, monkeys, rabbits, mice, rats, hamsters, and the like.

The time at which the drug against central nervous system inflammation of the present invention is administered is not limited, and may be suitably selected according to, for example, the dosage form, patient's age, severity of patient's symptoms, and the like. The dosage form is also not particularly limited, and a known dosage form may be employed. In particular, intravascular administration (e.g., intravenous administration) and oral administration are preferable.

The dosage of the drug against central nervous system inflammation of the present invention can be suitably selected according to the patient's age, severity of patient's symptoms, other conditions, and the like. The amount of the plasmalogen in the drug is preferably set within a range of 1 to 1,000 mg, and more preferably 10 to 100 mg, per day for an adult. The administration may be performed once, or performed multiple separate times (preferably, 2 to 3 times), per day.

When the drug against central nervous system inflammation of the present invention is used as a food additive, the drug (hereinafter, sometimes referred to as "food additive of the present invention") may consist only of the plasmalogen, or may be a food additive (i.e., a plasmalogen-containing composition for addition to food) suitably containing the plasmalogen, food-hygienically acceptable bases, carriers, additives, as well as other components and materials that can be used as food additives. Examples of the forms of such food additives include, but are not limited to, liquid, powder, flaky, granular, and paste forms. Specific examples thereof include seasonings (e.g., soy sauce, Worcestershire sauce, ketchup, and dressing), flakes (*furikake* [seasoning mix for sprinkling over cooked rice]), yakiniku [Korean-style barbecue] sauce, spices, paste-like roux (e.g., paste-like curry roux), and the like. These food additives can be appropriately prepared according to a known method. The amount of the plasmalogen contained in the food additive of the present invention is not particularly limited, as long as the central nervous system inflammation prevention effect is exerted, and is preferably 0.0005 to 100 mass%, more preferably 0.005 to 90 mass%, and still more preferably 0.05 to 80 mass%.

Such a food additive of the present invention is ingested when a food product containing the food additive is eaten. The addition of the food additive of the present invention to a food product may be conducted while a food product is being cooked or produced, or may be conducted immediately before or while a cooked food product is eaten. As the food additive is orally ingested as described above, the effect of alleviating central nervous system inflammation, and an effect of preventing or alleviating the symptoms of, for example, the diseases exemplified above are exerted. The intake amount of the food additive of the present invention, the subject receiving the food additive of the present invention, measurement of the content of plasmalogen, and the like, are preferably the same as, for example, those employed for the above-described pharmaceutical agent of the present invention.

When the drug against central nervous system inflammation of the present invention is used as food or drink, such a drug (hereinafter sometimes referred to as "food or drink of the present invention") is a composition (i.e., plasmalogen-containing food composition) suitably containing the plasmalogen, food-hygienically acceptable bases, carriers, additives, as well as other components, materials, and the like, that may be used as food. Examples thereof include those that contain a plasmalogen and that are used for alleviating central nervous system inflammation or for preventing or alleviating the symptoms of, for example, the diseases exemplified above, such as processed foods, beverages, health foods (e.g., foods with nutrient function claim and foods for specified health uses), supplements, medical foods (e.g., hospital diet, sick diet, and nursing-care food), and the like. When the plasmalogen contained in the drug is extracted from a biological tissue of livestock or poultry (e.g., cow, pig, chicken), the drug preferably serves as, but is not particularly limited to, for example, plasmalogen-containing processed flesh foods, such as hamburger, meatball, Vienna sausage, chicken soboro, chicken skin chips, etc.; plasmalogen-containing health foods containing processed flesh foods (e.g., foods with nutrient function claims, foods for specified health uses); plasmalogen-containing supplements; plasmalogen-containing medical foods; and the like. In addition, it is also possible to form the plasmalogen into, for example, a powder, to add to various food or drink products such as beverages (e.g., juice), snacks (e.g., chewing gums, chocolate, candies, biscuits, cookies, okaki and sembei [types of rice crackers], puddings, and Chinese-style almond jelly), bread, soups (including powdered soups, etc.), and processed foods.

When the food and drink of the present invention are prepared as health foods (e.g., foods with nutrient function claims or foods for specified health use, etc.) or a supplement, the forms thereof are preferably granules, capsules, pills (including, for example, chewable tablets), and beverages (drink preparations) in view of ease of continuous intake. Of these, in terms of ease of intake, forms such as capsules, tablets, and pills are preferable, but not particularly limited thereto. The drug against central nervous system inflammation as the food and drink of the present invention in the form of a granule, a capsule, a pill, or the like, can be appropriately prepared according to a hitherto known method using pharmaceutically and/or food-hygienically acceptable carriers and the like. When forming into other forms, a hitherto known method may also be employed.

The amount of the plasmalogen contained in the food and drink of the present invention is not particularly limited as long as an effect of alleviating central nervous system inflammation, or an effect of preventing or alleviating the symptoms of, for example, the diseases exemplified above can be exerted. The amount thereof is preferably 0.0005 to 100 mass%, more preferably 0.005 to 90 mass%, and still more preferably 0.05 to 80 mass%.

The food and drink of the present invention can be favorably used for alleviating central nervous system inflammation, and for preventing or alleviating the symptoms of, for example, the diseases exemplified above. The intake amount, the subject receiving the food and drink of the present invention, measurement of the content of plasmalogen, and the like, are preferably the same as, for example, those employed for the pharmaceutical agent of the present invention.

A hospital diet is a meal provided to people admitted to a hospital, a sick diet is a meal for the sick, and nursing-care food is a meal for people receiving care. The food and drink of the present invention are favorably used particularly as hospital diets, sick diets, or nursing-care food, in particular, for patients who are admitted to hospitals due to the diseases exemplified above or who are recuperating therefrom at home, or for patients receiving nursing care. It is also possible for people having a high possibility of developing the diseases exemplified above, such as elderly people, etc., to preventatively ingest the food or drink.

The present invention also provides a method for treating central nervous system inflammation, comprising administering (in particular, intravascularly or orally administering) an effective amount of the drug against central nervous system inflammation of the present invention to a subject suffering from central nervous system inflammation. The present invention also provides a method for preventing or treating neurodegenerative diseases and mental disorders, comprising administering (in particular, intravascularly or orally administering) an effective amount of the drug against central nervous system inflammation of the present invention to a subject suffering from a neurodegenerative disease or a mental disorder, or a patient having a high possibility of developing these diseases. Specifically, these methods can be performed through administration of the above-described drug against central nervous system inflammation of the present invention. In these methods, each of the conditions, such as the subject and intake amount, are as described above.

### Examples

The present invention is more specifically described below; however, the present invention is not limited to the following Examples.

### Preparation Example 1: Production of a Fraction Containing

### Biological Tissue-Extracted Plasmalogen

Chicken skin, which is an avian tissue, was collected in accordance with a hitherto known method, chopped into minced meat about 8 mm in size, then heated and compressed using a squeezer to remove neutral lipid, thereby preparing defatted chicken skin. The defatted chicken skin was then freeze-dried and ground by a usual method, thereby obtaining defatted chicken skin dry powder. The defatted chicken skin dry powder was hermetically stored together with a deoxidant until it was used for extraction.

### ■ Organic Solvent Extraction Step

### Step (1)

2 L of ethanol was added to 1 kg of the chicken-skin dry powder obtained above, and the resulting solution was stirred for 12 hours at 40°C and left still. Thereafter, the extraction solution was separated from the solid. 2 L of ethanol was added to the solid, and the extraction was performed again in the same method. All of the obtained extraction solutions were combined and filtered using filter paper, and dried under reduced pressure to obtain a condensed dry solid extraction.

### Step (2)

8 mL of water was added to the dry solid extraction. The mixture was stirred and centrifuged, and the upper layer was removed. 200 mL of acetone was added to the obtained precipitate, and the mixture was stirred and centrifuged at 4°C to remove the acetone layer. Then, 100 mL of acetone was added to the obtained precipitate, and the mixture was stirred and centrifuged to recover a precipitate. Next, 100 mL of a hexane/acetone (7:3) mixed solvent was added to the obtained precipitate, and the mixture was stirred and centrifuged to collect 20 g of a liquid layer (plasmalogen containing fraction). The liquid layer was immediately condensed and dried into a solid using a rotary evaporator. The centrifugations were conducted at 3000 rpm for 10 minutes, at 4°C for the group using only acetone or a hexane/acetone mixed solvent, and at 15°C for the rest.

### Step (3)

20 g of the plasmalogen-containing fraction obtained above was dispersed in 400 mL of a phospholipase A1 (Mitsubishi-Kagaku Foods Corporation) solution (10 mg/mL; 0.1 M citric acid-HCl buffer), and the mixture was stirred for 2 hours at 50°C under nitrogen gas. Then, the mixture was cooled, and hexane was added thereto at a volume twice that of the mixture. The mixture was stirred and partitioned twice, and the upper layer was collected to be condensed and dried into a solid. Next, 60 mL of acetone was added to the dry solid; and stirring, centrifuging, and precipitate-recovering were performed twice. Then, 60 mL of hexane/acetone (7:3) was added to the precipitate, and the mixture was stirred and centrifuged to recover a liquid layer (highly pure plasmalogen-containing fraction). After the liquid layer was condensed and dried into a solid, 240 mL of hexane/acetone (1:1) was added, and the mixture was transferred to a separatory funnel, 36 mL of water was added thereto, and stirring and partitioning were performed. The lower layer was removed, 96 mL of acetone/water (5:3) was added to the upper layer, and stirring and partitioning were again performed. The upper layer was collected, and immediately dried under reduced pressure to obtain a chicken skin-derived highly pure plasmalogen-containing substance.

### ■ Examination of Purity of Chicken Skin-Derived Highly Pure Plasmalogen-Containing Fraction

The chicken skin-derived highly pure plasmalogen-containing fraction obtained above was analyzed using an HPLC with the following conditions, thereby obtaining a chromatogram.

### ■ HPLC Analysis Conditions

Instrument; Shimadzu LC-10AD
Column; LiChrospher Diol 100 (250-4, Merck Inc.) (no pre-columns)
Solvent; Liquid A: hexane/2-propanol/acetic acid (82:17:1. v/v),
Liquid B: 2-propanol/water/acetic acid (85:14:1, v/v)+0.2% triethylamine

### Gradient Conditions:

**Table 1**

| Time (minute) | Liquid A | Liquid B |
|---|---|---|
| 0 → 1 | 95% | 5% |
| 1 → 24 | 95 → 60% | 5 → 40% |
| 24 → 25.5 | 60% | 40% |
| 25.5 → 28 | 60 → 100% | 0% |

Detection; ELSD evaporative light scattering detector (Shimadzu Corporation, Kyoto, Japan)

Fig. 1 shows the results. As shown therein, the results revealed that a chicken skin-derived plasmalogen is a mixture of ethanolamine plasmalogen (plPE) and choline plasmalogen (plPC).
Assuming that the area of the detected peak is 100% in total, the area ratio of plasmalogen was 94.6% in which plPE and plPC were 63.1% and 31.5%, respectively. It was thus revealed that the resulting chicken skin-derived highly pure plasmalogen-containing fraction contained 94.6 mass% of plasmalogen. It was also revealed that the mass ratio of plPE to plPC was about 2:1. In the experiment below, the chicken skin-derived highly pure plasmalogen-containing fraction was used as an avian tissue-derived plasmalogen.

Chicken breast meat was used as an avian tissue instead of chicken skin, and a chicken breast meat-derived highly pure plasmalogen-containing fraction was obtained in the same manner as above. The obtained fraction was analyzed using an HPLC in the same manner as above. Assuming that the area of the detected peak is 100% in total, the area ratio of plasmalogen was 94.6% in which plPE and plPC were 47.9% and 46.7%, respectively. It was thus revealed that the resulting chicken breast meat-derived highly pure plasmalogen-containing fraction contained 94.6 mass% of plasmalogen. It was also revealed that the mass ratio of plPE to plPC was about 1:1.

### Example 1: Examination of central inflammation prevention effect of a plasmalogen extracted from a biological tissue

The following experiment was performed for examining the influence of the avian-tissue extracted plasmalogen on central nerve inflammation.

### ■ Preparation of model mouse and administration of test substance

Cerebral inflammation model mice were created in the same manner as in Non-patent Document 2 (Lee JW et al., Neuroinflammation. 2008 Aug 29; 5:37). More specifically, LPS (lipopolysaccharide) as a stimulant of TLR4 (Toll-like receptor 4) was intraperitoneally administered to the mice for consecutive days, thereby creating chronic cerebral inflammation model mice.

Specifically, 4 male C57BL mice (Kyudo Co., Ltd.), aged 8-12 weeks, were intraperitoneally administered with LPS (250 µg/kg, Sigma Aldrich) once a day for seven consecutive days, thereby obtaining cerebral inflammation model mice. A control group consisting of four mice (the same male C57BL mice as above) was made by intraperitoneally administering physiological saline solution (Saline) (10 ml/kg) instead of LPS for seven consecutive days. Further, a test group consisting of four mice (the same male C57BL mice as above) was made by, in addition to the above procedure to create cerebral inflammation model mice, intraperitoneally administering an avian tissue extract plasmalogen (20 mg/kg) in the same manner as the LPS administration 30 minutes before the LPS administration for seven consecutive days.

### ■ Immunostaining

### Preparation of brain section

Each mouse was anesthetized with pentobarbital 24 hours after the final LPS administration (in the control group, 24 hours after the final physiological saline solution administration). After the laparotomy, blood was removed by passing a 0.9% physiological saline solution through the left ventricle. Thereafter, the brain was perfused-fixed using 4% para formaldehyde. The isolated brain was placed still overnight using 4% para formaldehyde, and further placed still using a 30% sucrose solution. Thereafter, a 30-40 µm brain cryosection was created using a cryostat.

### ■ Immunostaining of glial cells

The brain cryosection obtained above was treated with a blocking solution for 30 minutes at room temperature, reacted with the primary antibody appropriately diluted, and then placed still overnight at 4°C. After washing it several times with PBS (phosphate buffer physiological saline solution), the brain section was reacted with the secondary antibody appropriately diluted (1:500) for 6 hours at room temperature. The brain section was then washed with PBS several times, thereby obtaining a brain section sample. A confocal microscope image of the sample was obtained. Fig. 2(a) shows images of the cerebral cortex (Cortex), Fig. 3(a) shows images of the dentate gyrus (DG), and Fig. 4(a) shows images of the hippocampus (CA1).

The primary antibody was an anti-Iba-1 (ionized calcium-binding adapter molecule-1: microglial cell marker) antibody (Wako Chemicals). The secondary antibody was a labeled anti-rabbit IgG antibody (Alexa Fluor 488 (Molecular Probes)). Further, a fluorescently labeled anti-GFAP (glial fibrillary acidic protein; astrocyte marker) antibody (Sigma Aldrich) was used for the immunostaining of astrocyte. Therefore, the secondary antibody reaction step was not necessary in the experiment using the anti-GFAP antibody.

Microglia and astrocytes are glial cells. Microglial cells undergo cell growth or structural change as a reaction to brain damage or inflammation, and are considered to control immunity in the central nervous system and to be deeply related to various neurodegenerative disorders or inflammation. Astrocytes are considered to retain nerve fibers and the like.

### ■ Immunostaining of Aβ protein and nerve cell nucleus

The brain section obtained above was heat-treated using a citric acid buffer, reacted with the primary antibody appropriately diluted, and then placed still for 18 hours at room temperature. After washing it several times with PBS (phosphate buffer physiological saline solution), the brain section was reacted with the secondary antibody appropriately diluted (1:250) for 6 hours at room temperature. The brain section was then washed with PBS several times, thereby obtaining a brain section sample. Confocal microscope images of the sample were obtained. Fig. 5 shows images of the cerebral cortex (Cortex), and Fig. 6 shows images of the hippocampus (CA1).

In the immnunostaining of Aβ protein, the primary antibody was an antibody (ab14220, Abcam) that recognizes Aβ1-16. The secondary antibody was a labeled anti-rabbit IgG antibody (Alexa Fluor 488 (Molecular Probes)). In the immunostaining of nerve cell nucleus, the primary antibody was a mouse-derived anti-neuronal nuclei (NeuN) monoclonal antibody (Millipore) that recognizes nerve cell nucleus, and the secondary antibody was a goat-derived fluorescently labeled anti-mouse IgG antibody (Alexa Fluor 568 (Molecular Probes)).

In the above examination using immunostaining, a 10-fold diluted Block Ace (DS Pharma Biomedical Co., Ltd.) was used to dilute the primary and secondary antibodies.

### ■ Analysis of number of microglial cells and astrocyte cells

Recent studies suggested that activated glial cells cause inflammation and thereby damage peripheral nerve cells. In particular, in Alzheimer's disease or Parkinson's disease, glial cells in the vicinity of degenerative nerve cells are assumed to cause inflammation by producing inflammatory cytokine, thereby causing nerve degeneration.

Therefore, the number of glial cells in the images (Fig. 2(a), Fig. 3(a), and Fig. 4(a)) obtained above was counted. More specifically, the numbers of Iba-1-positive cells and GFAP-positive cells (i.e., fluorescing cells) in a 40000 µm² (200 µm x 200 pm) area in each image were counted. Statistical significant difference examination was performed using Fisher's PLSD based on one-way ANOVA.

The results are shown in Fig. 2(b) (cerebral cortex microglial cell), Fig. 2(c) (cerebral cortex astrocyte cells), Fig. 3(b) (dentate gyrus microglial cells), Fig. 3(c) (dentate gyrus astrocyte cells), Fig. 4(b) (hippocampus microglial cells) and Fig. 4(c) (hippocampus astrocyte cells). In these figures, "*" denotes p<5%, "**" denotes p<1%, and "40³ µm²" denotes "40000 µm²".

The results of Figs. 2 to 4 revealed that LPS administration for 7 consecutive days significantly increased the numbers of glial cells (microglial cells and astrocyte cells) in the cerebral cortex (Cortex), dentate gyrus (DG), and hippocampus (CA1). It was further revealed that administration of plasmalogen significantly suppressed the increase in glial cells.

The results suggested that the LPS administration increased the number of glial cells and activated glial cells, thereby causing cerebral inflammation, and that the administration of plasmalogen suppressed the increase in glial cells, thereby alleviating the inflammation.

Further, the results of Fig.5 and Fig.6 revealed that LPS administration for 7 consecutive days caused accumulation of amyloid beta (Aβ) protein in cerebral cortex (Cortex) and hippocampus (CA1). It was further revealed that administration of plasmalogen significantly suppressed the accumulation of amyloid beta (Aβ) protein.

The accumulation of amyloid beta (Aβ) protein is assumed to be one of major causes of Alzheimer's disease. Further, it is also known that Aβ protein directly damages nerve cells. Based on the above finding that the administration of plasmalogen alleviates or treats inflammation, and also suppresses the accumulation of Aβ protein, it was concluded that plasmalogen was particularly effective for the prevention and treatment of neurodegenerative diseases such as Alzheimer's disease.

## Claims

1. A drug against central nervous system inflammation containing a plasmalogen.

2. The drug against central nervous system inflammation according to claim 1, containing a plasmalogen extracted from a biological tissue.

3. The drug against central nervous system inflammation according to claim 2, containing a plasmalogen extracted from an avian tissue.

4. The drug against central nervous system inflammation according to any one of claims 1 to 3, wherein the plasmalogen includes an ethanolamine plasmalogen and a choline plasmalogen.

5. The drug against central nervous system inflammation according to claim 4, wherein 90 mass% or more of the plasmalogen is an ethanolamine plasmalogen and a choline plasmalogen.

6. The drug against central nervous system inflammation according to claim 4 or 5, wherein a mass ratio of ethanolamine plasmalogen:choline plasmalogen in the plasmalogen is 1:5 to 5:1.

7. The drug against central nervous system inflammation according to any one of claims 1 to 6, utilized for preventing or treating at least one type of disease selected from the group consisting of dementia, Parkinson's disease, depression, and schizophrenia.
